# EUROPÄISCHE PATENTANMELDUNG

(11) **EP 0 822 251 A2**
(43) Veröffentlichungstag der Anmeldung: **04.02.1998**
(21) Anmeldenummer: 97890138.7
(22) Anmeldetag: 16.07.1997
(51) Int. Cl.: C12M 1/107

(54) **Vorrichtung zur Gewinnung von Biogas**

(30) Priorität: 01.08.1996 AT 1380/96
(71) Anmelder: Falle, Ignaz, 5071 Wals-Himmelreich (AT)
(72) Erfinder: Falle, Ignaz, 5071 Wals-Himmelreich (AT)
(74) Vertreter: Puchberger, Rolf, Dipl. Ing.

(57) **Zusammenfassung**

Die Erfindung betrifft eine Vorrichtung zur Gewinnung von Biogas aus Gärsubstrat, insbesondere Gülle und biogenen Abfällen, wobei die Vcrrichtung einen Fermenter und einen Nachgärbehälter mit Gasspeicher umfaßt, wobei der Fermenter vertikal in einen Hauptgärraum und einen Nebengärraum unterteilt ist, und das frische Gärsubstrat in den Hauptgärraum eingeleitet ist, und der Hauptgärraum und Nebengärraum im Bodenbereich des Fermenters miteinander verbunden sind, dadurch gekennzeichnet, daß das frische Gärsubstrat von oben in den Hauptgärraum (9) eingeleitet ist, Hauptgärraum (9) und Nebengärraum (10) im Bodenbereich über Mischöffnungen (12) verbunden sind, daß die Gasräume (51, 52) des Hauptgärraumes (9) und Nebengärraumes (10) durch eine Gasüberströmleitung (53) mit einem Gasventil (14) verbunden sind, wobei das Gasventil (14) alternierend und in Abhängigkeit vom Gasdruck geöffnet und geschlossen wird, und daß vom Nebengärraum (10) ein Fermenterablauf (44) zum Nachgärbehälter (19) führt.

## Beschreibung

Die Erfindung betrifft eine Vorrichtung zur Gewinnung von Biogas aus Gärsubstrat, insbesondere Gülle und biogenen Abfällen, wobei die Vorrichtung einen Fermenter und einen Nachgärbehälter mit Gasspeicher umfaßt, wobei der Fermenter vertikal in einen Hauptgärraum und einen Nebengärraum unterteilt ist, und das frische Gärsubstrat in den Hauptgärraum eingeleitet ist, und der Hauptgärraum und Nebengärraum im Bodenbereich des Fermenters miteinander verbunden sind. Weiters betrifft die Erfindung ein Verfahren zur Biogasgewinnung.

Die bekannten Biogas-Anlagen haben vielfach den Nachteil, daß sie im Aufbau und im Betrieb kompliziert sind und damit hohe Investitionen erfordern. Die Verwendung mechanischer Rührwerke im Fermenter ist energieaufwendig und erfordert ständige Wartung. Die Gasausbeute ist dabei oft ungenügend.

Bei herkömmlichen einfach gehaltenen und billigen Biogas-Anlagen ist die Gasausbeute zumeist schlecht und es fehlt die Gasentschwefelung.

Die DE 34 27 976 A1 zeigt eine Biogasanlae der eingangs beschriebenen Type, wobei eine Heizeinrichtung im Hauptgärraum die Gülle erwärmt und thermisch zirkulieren läßt. Die Gülle gelangt kontinuierlich über einen Ringspalt im Bodenbereich in einen ringförmigen Abschnitt des Nebengärraumes, aus dem die Gülle durch Einleiten von Biogas herausgedrückt und in Zirkulation gehalten wird. Dieser bekannte Aufbau ist kompliziert und schwierig zu steuern.

Demgegenüber zeigt die DE 31 39 914 A1 eine Biogasanlage, die sehr einfach aufgebaut ist, jedoch eine schlechte Durchmischung der Gülle und eine niedrige Gasausbeute hat.

Gemäß vorliegender Erfindung soll eine Vorrichtung zur Gewinnung von Biogas geschaffen werden, die bei niedrigen Kosten eine einfache Konstruktion vorsieht, wobei eine optimale Gasausbeute bei guter Gasqualität erzielt wird. Auf mechanische Rührwerke im Fermenter soll verzichtet werden.

Die erfindungsgemäße Vorrichtung ist dadurch gekennzeichnet, daß das frische Gärsubstrat von oben in den Hauptgärraum eingeleitet ist, Hauptgärraum und Nebengärraum im Bodenbereich über Mischöffnungen verbunden sind, daß die Gasräume des Hauptgärraumes und Nebengärraumes durch eine Gasüberströmleitung mit einem Gasventil verbunden sind, wobei das Gasventil alternierend und in Abhängigkeit vom Gasdruck geöffnet und geschlossen wird, und daß vom Nebengärraum ein Fermenterablauf zum Nachgärbehälter führt.

Weitere vorteilhafte Merkmale der Erfindung sind den Patentansprüchen, der nachfolgenden Beschreibung und den Zeichnungen zu entnehmen.

Im folgenden wird die Erfindung anhand eines Ausführungsbeispieles näher erläutert.

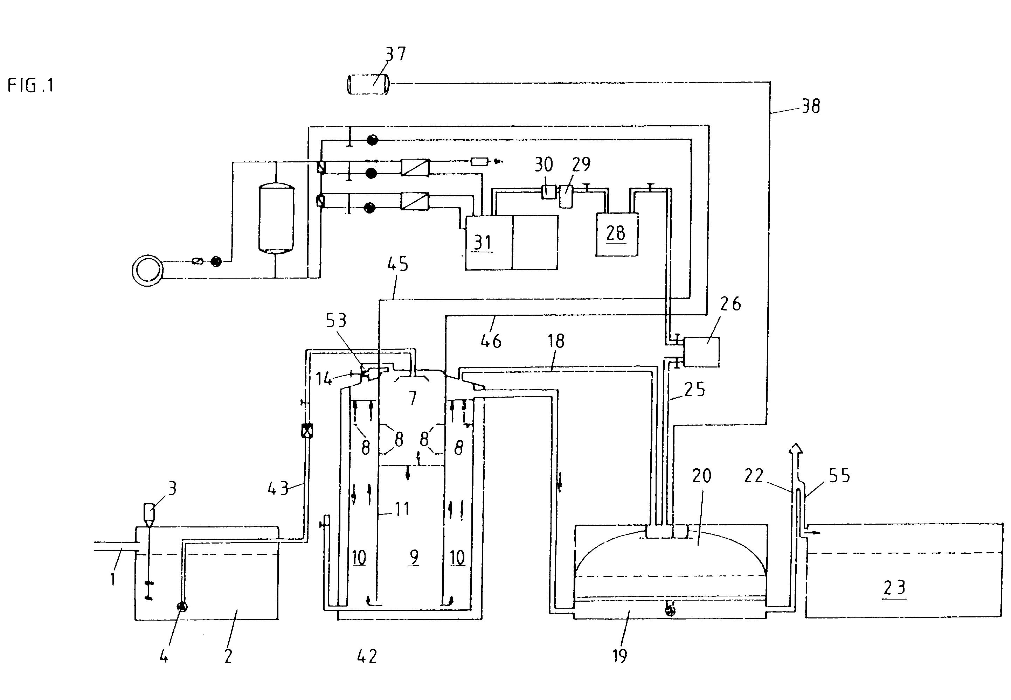

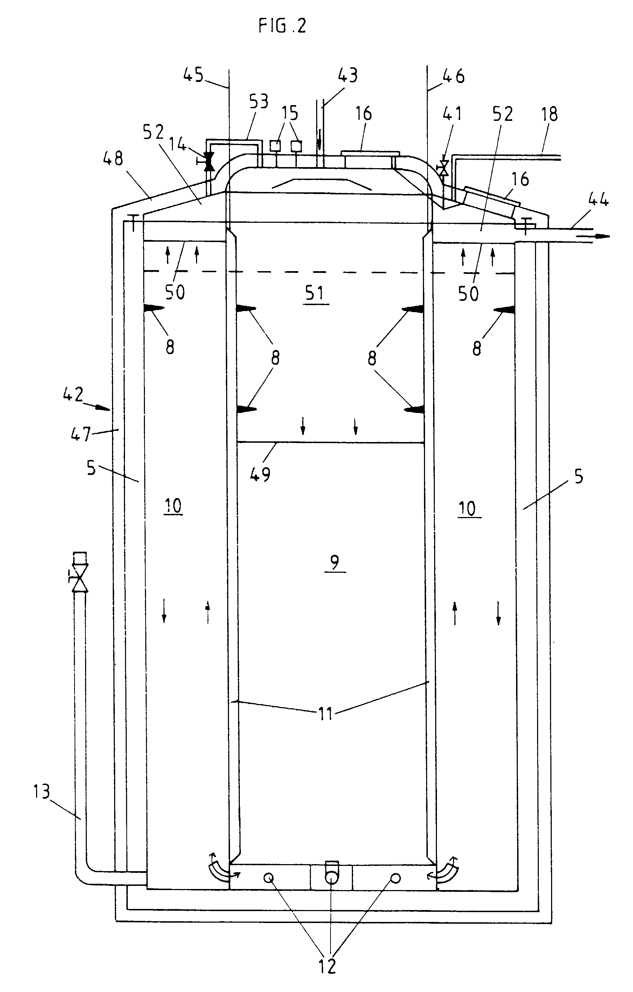

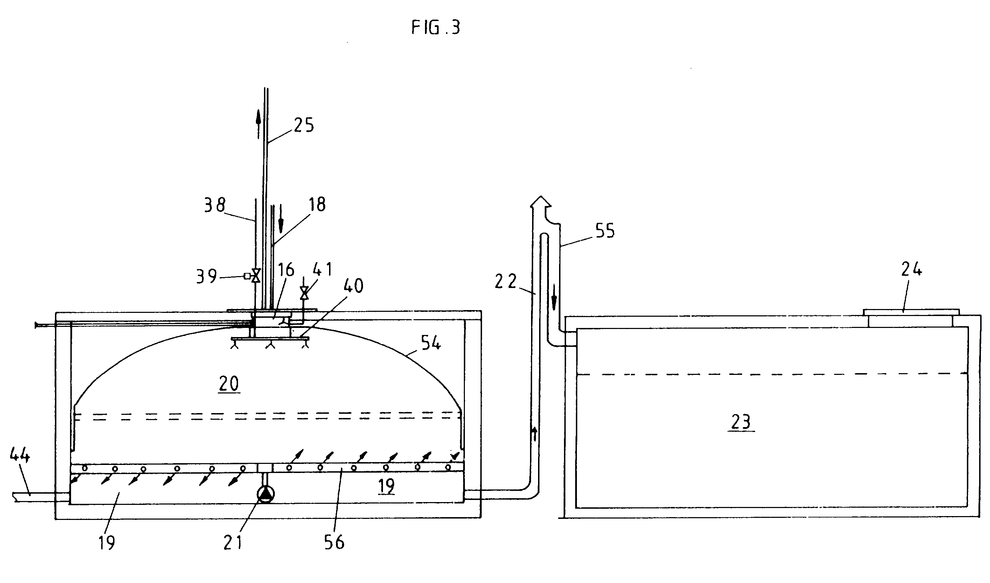

Die Fig.1 zeigt schematisch eine erfindungsgemäße BiogasAnlage mit den zugehörigen Geräten und Anlagen zur Energieumwandlung und Energieerzeugung. Die Fig. 2 und 3 zeigen in vergrößertem Maßstab schematische Querschnittsdarstellungen einzelner Teile der Biogasanlage.

Die in Fig.1 dargestellte Anlage umfaßt im wesentlichen eine Mischkammer 2, einen Fermenter 42, einen Nachgärungsbehälter 19 mit darüber liegendem Gasbehälter 20 und das Biogülle-Endlager 23.

Durch den Einlauf 1 gelangen die Gülle, Stallmist, Fäkalien und organischer Müll in die Mischkammer 2 zur Aufbereitung. Der Mixer 3 vermischt die organischen Abfälle und hält sie in Bewegung. Der Inhalt wird als Gärsubstrat von der Güllepumpe 4, die mit einem Schneidwerk versehen sein kann, über die Fermenterzulaufleitung 43 dem Fermenter 42 von oben zugeführt. Im Fermenter wird das Gärsubstrat weitgehend ausfermentiert und über den Fermenterablauf 44 in den Nachgärungsbehälter 19 eingespeist. Über die Gasleitung 18 gelangt das im Fermenter 42 gebildete Biogas ebenfalls in den Nachgärungsbehälter 19 bzw. in den darüber liegenden Gasbehälter 20. Über die Heizleitungen 45,46 wird der Fermenter 42 beheizt.

Vom Gasspeicher 20 wird das Biogas über die Gasableitung 25 über einen Kondenswasserabscheider 26, einen Gaszähler 28, sowie Gasfilter 29 und Druckminderer 30 einem Blockheizkraftwerk 31 zugeleitet. Druckluft vom Luftkompressor 37 gelangt über die Luftleitung 38 in den Gasbehälter 20.

Die im Nachgärungsbehälter 19 ausfermentierte Gülle gelangt über ein Sicherheitsstandrohr 22 in das Biogülle-Endlager 23.

Der Aufbau des Fermenters 42 und dessen Wirkungsweise wird an Hand der Fig.2 näher erläutert.

Der Fermenter 42 besteht aus einer zylinderförmigen Betonwanne 5, die aussen durch die Isolierung 47 isoliert ist. Oben ist der Fermenter durch den Fermenterdeckel 48 dicht abgeschlossen. Im Fermenter steht ein beheizter Doppelmantelzylinder 11, der eine Trennwand bildet und zum Fermenterdeckel 48 abgedichtet ist. Somit ist der Fermenter 42 in eine Hauptgärkammer 9 und eine aussen liegende Nachgärkammer 10 geteilt. Durch Mischöffnungen 12 sind die beiden Gärkammern miteinander im Bodenbereich des Fermenters verbunden. Der Doppelmantelzylinder 11 sieht eine große Heizfläche vor, sodaß diese mit niedriger Temperatur bis etwa 60° C betrieben werden kann. Dadurch ist eine schonende Erwärmung des Gärsubstrates gewährleistet, was für die Gährung vorteilhaft ist.

Über den Füllspiegeln 49,50 des Gärsubstrates sammelt sich das fermentierte Biogas in den Gasräumen 51 und 52. Die Gasräume 51 und 52 sind durch eine Gasüberströmleitung 53, die ein Überströmventil 14 aufweist, verbunden. Das Überströmventil 14 wird von Druckschaltern 15 gesteuert.

Über das Abzugsrohr 13 kann der Grundschlamm des Fermenters abgezogen werden. Durch die Einstiegsöffnungen 16 kann der Fermenter betreten und optisch kontrolliert werden. Mit 41 ist eine Wassereinspritzdüse bezeichnet, mit der die Einstiegsöffnungen 16 gereinigt werden können. Wenn sie aus durchsichtigem Material bestehen, kann somit eine optische Kontrolle des Fermenters durch die Einstiegsöffnungen auch bei geschlossenem Zustand erfolgen.

Der Abzug des im Fermenter gebildeten Gases erfolgt über die Gasleitung 18 aus dem Gasraum 52 der Nachgärkammer 10.

Vom ringförmigen Gasraum 52 der Nachgärkammer 10 geht weiters die Überlaufleitung 44 ab, die das fermentierte Gärsubstrat in den Bodenbereich des Nachgärungsbehälters 19 einleitet (Fig.3). Die Mischpumpe 21 bewirkt gegebenenfalls die erforderliche Durchmischung der Gülle im Nachgärungsbehälter.

Der Gasbehälter 20 wird durch eine Folie 54 gebildet. Im Bereich der Einstiegsöffnung 16 ist ein Luftverteilungsrohr 40 mit Luftverteilungsdüsen vorgesehen, durch die über das Luftventil 39 und die Luftzuleitung 38 Luft zur Entschwefelung des Biogases eingeleitet werden kann. Die Wassereinspritzdüse 41 dient der Reinigung eines etwaigen Fensters in der Einstiegsöffnung 16.

Vom Nachgärungsbehälter 19 geht ebenfalls im Bodenbereich das Sicherheitsstandrohr 22 ab, von dem das Überlaufrohr 55 abzweigt und in das Biogülle-Endlager 23 führt. Mit 24 ist die Grubenabdeckung des Endlagers bezeichnet.

Im folgenden wird die Funktionsweise der erfindungsgemäßen Vorrichtung beschrieben.

Das gut vorbereitete Gärsubstrat aus Gülle, biogenen Abfällen etc. wird von der Güllepumpe 4, gegebenenfalls zerkleinert, über die Fermenterzulaufleitung 43 in die Hauptgärkammer 9 des Fermenters 42 eingeleitet. Zur besseren Verteilung des eingeleiteten Gärsubstrates ist im Einlaufbereich eine Verteilerplatte 7 vorgesehen. Die Prallplatten 8 an der Innenseite des Doppelmantelzylinders 11 dienen der besseren Durchmischung des vorbeiströmenden Gärsubstrates.

In der Hauptgärkammer 9 kommt es, auch unter Einfluß der Wärme des beheizten Doppelmantelzylinders 11, zur Bildung des Biogases, welches sich im Gasraum 51 sammelt und dort einen Druck aufbaut. Der Füllspiegel 49 des Gärsubstrates wandert nach unten, wobei das verdrängte Gärsubstratvolumen durch die Mischöffnungen 12 in den Nachgärraum 10 verdrängt wird. Dort wird überfließendes Gärsubstrat durch den Fermenterablauf 44 abgezogen.

Die Druckschalter 15 schalten bei Erreichen eines vorgegebenen Druckes im Gasraum 51 das Überströmventil 14 frei, sodaß ein Druckausgleich zwischen dem Gasraum 51 der Hauptgärkammer 9 und dem Gasraum 52 der Nachgärkammer 10 erfolgt. Dabei kommt es zu einem relativ raschen Druckabfall im Gasraum 51, wodurch sich der Füllspiegel 49 ebenfalls rasch nach oben und kommunizierend der Füllspiegel 50 in der Nachgärkammer 10 nach unten bewegen. In beiden Gärkammern bewirken die Prallplatten 8 und die Mischöffnungen 12 eine gute Durchmischung des vorbeiströmenden Gärsubstrates.

Sobald der Druckausgleich hergestellt ist, wird das Überströmventil 14 geschlossen, in der Hauptgärkammer 9 bildet sich wieder erhöhter Druck aus und der Kreislauf beginnt von neuem.

Die Zuleitung des Gärsubstrates über die Fermenterzulaufleitung 43 kann kontinuierlich oder diskontinuierlich erfolgen. Das Rückschlagventil 6 verhindert ein Zurückfließen des Substrates bei den auftretenden Druckschwankungen.

Im Nachgärraum 10 kommt es zusätzlich zu einer Vermischung dadurch, das der Doppelmantelzylinder 11 eine höhere Temperatur aufweist, als die Betonwand 5. Durch Pfeile sind die zirkulierenden Ströme angedeutet.

Durch die ständige Bewegung des Gärsubstrates kommt es zu einem hohen Fermentierungsgrad und Ausgasung. Die Nachteile herkömmlicher Rührwerke und mechanischer Mischpumpen im Fermenter werden vermieden.

Im Nachgärungsbehälter kommt es zum Ausgasen der Gasreste in der Gülle, wobei dieses Restgas gemeinsam mit dem vom Gasraum 52 über die Gasleitung 18 abgezogenen Gas im Gasbehälter 20 gesammelt wird. Im dargestellten Ausführungsbeispiel dient eine elastische Folie 54 als Behälter, die sich je nach Gasmenge aufbläht.

Die Durchmischung im Nachgärbehälter wird mittels Güllepumpe 21 und einem Düsenstrahlrohr 56 erzielt.

Die erforderliche Entschwefelung des im Gasbehälter 20 befindlichen Biogases wird dadurch erreicht, daß Luft über das Luftverteilerrohr 40 genau in dosierter Menge eingeleitet wird. Eine Luftmenge von 3 bis 4 %, bezogen auf das Biogas, ist ein günstiger Wert. Die Luftmenge wird über das Luftsteuerventil 39 eingestellt. Die Entschwefelung kann bis zu 95 % betragen. Der gebildete Schwefel fällt auf die darunterliegende Gülle auf und wird in diese eingemischt.

Die entgaste Biogülle wird über das Sicherheitsstandrohr 22 und das Überlaufrohr 55 dem Endlager 23 zugeleitet. Die Höhe des Sicherheitsstandrohres 22 respektive die Höhe des darin stehenden Güllespiegels bestimmt den Gasdruck der gesamten Anlage. Ein etwa erforderlicher Druckausgleich kann über dieses Sicherheitsstandrohr erfolgen.

Die verschiedenen Einstiegsöffnungen 16 in den verschiedenen Tanks können mit einem transparenten Deckel versehen sein, sodaß ohne Öffnen der Kammern eine optische Kontrolle möglich ist. Im Falle der Verschmutzung der Glasdeckel können sie durch die Wasserspritzdüsen 42 gereinigt werden.

Die Übersichtsdarstellung gemäß Fig.1 zeigt auch eine Anwendung für die weitere Nutzung des Biogases aus dem Gasbehälter 20. Es erfolgt eine Trocknung im Kondenswasserableiter 26 und weiters strömt das Gas durch den Gaszähler 28, Gasfilter 29 und Druckminderer 30 zum Blockheizkraftwerk 31. Dort kann sowohl kalorische Energie als auch elektrische Energie erzeugt werden. Über Wärmetauscher 34 vom Gasmotor und einen Abgaswärmetauscher 33 wird Abwärme rückgewonnen, wobei ein Pufferspeicher 35 für die Wärme vorgesehen ist. Mit 36 ist schematisch ein beliebiger Wärmeverbraucher wie zB. eine Heizanlage bezeichnet.

Ein Teil der erzeugten Wärme wird über die Heizleitungen 45,46 im Kreislauf den Heizregistern des Doppelmantelzylinders zugeleitet. Bevorzugt sind Temperaturen von etwa 35 bis 40° C in der Gülle.

Die vorliegende Erfindung ist auf das dargestellte Ausführungsbeispiel nicht beschränkt. Der Doppelmantelzylinder 11 muß nicht zylindrisch sein und auch der Fermenter 42 kann eine andere Form haben. Wesentlich ist, daß ein Hauptgärraum und ein Nachgärraum vorgesehen sind, die im unteren Bereich durch Öffnungen miteinander verbunden sind. Die Nutzung des Biogases kann selbstverständlich jede andere gewünschte Form haben, wie zB. Einspeisung in das öffentliche Gasnetz und dergl.

Das Verfahren zur Biogasgewinnung aus Gärsubstrat bestehend aus Gülle und anderen organischen Stoffen ist dadurch gekennzeichnet, daß das Gärsubstrat unter Erwärmung in einem Hauptgärraum und nachfolgend in einem Nebengärraum eines Fermenters fermentiert wird, daß das vom Nebengärraum abgezogene Biogas einem Gasspeicher zugeleitet wird und dem Biogas im Gasspeicher zur Entschwefelung Luft zugesetzt wird.

## Patentansprüche

1. Vorrichtung zur Gewinnung von Biogas aus Gärsubstrat, insbesondere Gülle und biogenen Abfällen, wobei die Vorrichtung einen Fermenter und einen Nachgärbehälter mit Gasspeicher umfaßt, wobei der Fermenter vertikal in einen Hauptgärraum und einen Nebengärraum unterteilt ist, und das frische Gärsubstrat in den Hauptgärraum eingeleitet ist, und der Hauptgärraum und Nebengärraum im Bodenbereich des Fermenters miteinander verbunden sind, dadurch gekennzeichnet, daß das frische Gärsubstrat von oben in den Hauptgärraum (9) eingeleitet ist, Hauptgärraum (9) und Nebengärraum (10) im Bodenbereich über Mischöffnungen (12) verbunden sind, daß die Gasräume (51, 52) des Hauptgärraumes (9) und Nebengärraumes (10) durch eine Gasüberströmleitung (53) mit einem Gasventil (14) verbunden sind, wobei das Gasventil (14) alternierend und in Abhängigkeit vom Gasdruck geöffnet und geschlossen wird, und daß vom Nebengärraum (10) ein Fermenterablauf (44) zum Nachgärbehälter (19) führt.

2. Vorrichtung nach Anspruch 1, dadurch gekennzeichnet, daß im Hauptgärraum (9) und im Nebengärraum (10) Prallplatten (8) vorgesehen sind, um ein Vermischen des Gärsubstrates zu verstärken.

3. Vorrichtung nach Anspruch 1 oder 2, dadurch gekennzeichnet, daß die Trennwände (Doppelmantelzylinder 11) zwischen Hauptgärraum (9) und Nebengärraum (10) beheizt sind.

4. Vorrichtung nach einem der Ansprüche 1 bis 3, dadurch gekennzeichnet, daß die Trennwände durch einen beheizten Doppelmantelzylinder (11) gebildet sind, der zum Fermenterdeckel (48) abgedichtet ist.

5. Vorrichtung nach einem der Ansprüche 1 bis 4, dadurch gekennzeichnet, daß das Gasventil (14) durch Druckschalter (15) gesteuert ist, wobei das Gasventil (14) bei Erreichen eines voreingestellten Gasdruckes geöffnet und bei Druckabfall wieder geschlossen wird.

6. Vorrichtung nach einem der Ansprüche 1 bis 5, dadurch gekennzeichnet, daß zur Ableitung des im Fermenter (42) gebildeten Gases eine vom Nebengärraum (10) abführende Gasleitung (18) vorgesehen ist.

7. Vorrichtung nach einem der Ansprüche 1 bis 6, dadurch gekennzeichnet, daß über dem Nachgärbehälter (19) der Gasspeicher (20) angeordnet ist und daß eine Luftzuleitung (38) in den Gasspeicher (20) zur Gasentschwefelung vorgesehen ist.

8. Vorrichtung nach Anspruch 7, dadurch gekennzeichnet, daß die Einleitung des Fermenterablaufes (44) im Bodenbereich des Nachgärbehälters (19) erfolgt, daß eine Mischvorrichtung (21,56) vorgesehen ist und daß zur Ableitung des entgasten Gärsubstrates ein Standrohr (22) vorgesehen ist, dessen Überlauf (55) mit dem Endlager (23) für die Gülle verbunden ist.

9. Verfahren zur Biogasgewinnung aus Gärsubstrat bestehend aus Gülle und anderen organischen Stoffen, wobei das Gärsubstrat unter Erwärmung in einem Hauptgärraum und nachfolgend in einem Nebengärraum eines Fermenters fermentiert wird, und das vom Nebengärraum abgezogene Biogas einem Gasspeicher zugeleitet wird, dadurch gekennzeichnet, daß dem Biogas im Gasspeicher zur Entschwefelung Luft zugesetzt wird.

10. Verfahren nach Anspruch 9, dadurch gekennzeichnet, daß die Luftmenge 3 bis 4 Vol.% bezogen auf die Menge des Biogases beträgt.
